# EUROPÄISCHE PATENTANMELDUNG

(11) **EP 0 626 604 A2**
(43) Veröffentlichungstag der Anmeldung: **30.11.1994**
(21) Anmeldenummer: 94107675.4
(22) Anmeldetag: 18.05.1994
(51) Int. Cl.: G02B 23/24, A61B 1/00, A61M 25/01

(54) **Flexibles Endoskoprohr**

(30) Priorität: 28.05.1993 DE 4317914
(71) Anmelder: CARL HAAS GmbH & CO., D-78713 Schramberg (DE)
(72) Erfinder: Carl, Günther, Ing., D-78730 Lauterbach (DE); Uihlein, Bernhard, Dipl.-Ing. (FH), D-72581 Dettingen (DE)
(74) Vertreter: Patentanwälte Westphal, Buchner, Mussgnug Neunert, Göhring

(57) **Zusammenfassung**

Flexibles Endoskoprohr, welches aus kurzen, rohrförmigen, durch Schwenkorgane 14, 16 in Längsrichtung des Rohres stabil miteinander verbundenen Gliedern (10) besteht und durch ein oder mehrere längs des Rohres verlaufende Zugseile (44) steuerbar ist. Die Schwenkorgane (14, 16) und Zugseile (44) sind dabei im Bereich jedes rohrförmigen Gliedes (10) innerhalb der Wandstärke des Gliedes angeordnet.

## Beschreibung

Die Erfindung betrifft ein flexibles Endoskoprohr nach dem Oberbegriff des Anspruchs 1.

Ein derartiges Endoskoprohr ist aus der DE 35 04 824 A1 bekannt, wobei die Schwenkachsen aufeinanderfolgender rohrförmiger Glieder entweder parallel zueinander liegen oder, um die Biegbarkeit des Endoskoprohres in zwei zueinander senkrechten Ebenen zu ermöglichen, abwechselnd aufeinander senkrecht stehen. Die gelenkige Verbindung zwischen den einzelnen Gliedern wird bei dem bekannten Endoskoprohr durch Nieten oder Hohlnieten hergestellt, die in entsprechenden, einander diametral gegenüberliegenden Bohrungen der rohrförmigen Glieder angebracht sind. Die die Niete unmittelbar aufnehmenden Bereiche der Einzelglieder sind dabei steg- oder lappenförmig ausgebildet und nach außen oder innen abgekröpft.

Dieser Aufbau des bekannten Endoskoprohres verlangt nicht nur einen verhältnismäßig großen Herstellungsaufwand, sondern hat auch einen nicht unerheblichen Platzbedarf, wobei der zur Verfügung stehende Freiraum innerhalb des Endoskoprohrs verkleinert oder dessen Außendurchmesser vergrößert wird. Beides hat erhebliche Nachteile.

Ein weiterer Nachteil besteht in der Art der Führung der Zugseile, mit denen die krümmbaren Abschnitte des Endoskoprohres gesteuert werden. Diese Zugseile laufen in aus der Wand der rohrförmigen Glieder nach innen gestülpten ösenartigen Bauteilen, die den im Innenraum des Rohres zur Verfügung stehenden Raum einschränken.

Durch die Erfindung soll ein flexibles Endoskoprohr geschaffen werden, das leicht herzustellen und zu montieren ist und insbesondere weder den im Innern des Rohres zur Verfügung stehenden Platz durch Scharniere, Ösen, Nieten, Achsen und dergl. wesentlich verkleinert noch den Außendurchmesser des Rohres durch diese Teile wesentlich vergrößert.

Diese Aufgabe wird erfindungsgemäß durch die Merkmale des Anspruchs 1 gelöst.

Durch die Anordnung sämtlicher Schwenkorgane und Zugseile innerhalb der Wandstärke der rohrförmigen Glieder läßt sich bei sehr einfacher Herstellung und Montage ein absolut behinderungsfreier Innenraum des Endoskoprohres erzielen, der infolgedessen für den Einbau optischer oder anderer Teile des Endoskops voll zur Verfügung steht.

Aus DE 26 18 732 B ist ein flexibles Endoskoprohr mit Schwenkorganen bekannt, deren einer Teil zwar innerhalb der Wandstärke des zugehörigen Gliedes liegt, wobei der jeweils andere Teil eines Schwenkorgans jedoch aus der Wandstärke des Gliedes heraus nach innen abgekröpft ist, so daß Innenraum des Endoskoprohrs verlorengeht. Von den abgekröpften Teilen der Schwenkorgane ragen sog. Drahthalter noch weiter ins Innere des Endoskoprohrs, in denen die Zugdrähte oder Zugseile geführt sind. Dadurch wird der zur Verfügung stehende Innenraum des Endoskoprohrs insgesamt stark beeinträchtigt.

US 3,788,304 zeigt ein flexibles Endoskoprohr mit ebenfalls aus der Wandstärke der einzelnen Glieder nach außen und innen abgekröpften Schwenkorganteilen. Die Zugseile sind bei diesem bekannten Endoskoprohr in Rillen geführt, deren Wände ebenfalls aus der Wandstärke des jeweiligen Gliedes heraus ins Innere des Endoskoprohrs vorstehen.

Die Unteransprüche betreffen vorteilhafte Ausgestaltungen der Erfindung.

Die besonders einfache Ausbildung der paarweise gegenüberliegenden Schwenkorgane als Gleitgelenke gemäß Anspruch 2, beispielsweise in Form von Kugel-, Zylinder- oder Kegelgelenken, gestattet eine hervorragende Festlegung der einzelnen Glieder des Rohres gegen Torsion sowie in Längsrichtung des Rohres bei besonders einfacher Herstellung.

Die axiale Festlegung der Schwenkorgane, d.h. die Festlegung in Richtung der Schwenkachse, erfolgt in besonders einfacher Weise durch Verjüngung der Gleitflächen der gegenüberliegenden Schwenkorgane in entgegengesetzter Richtung gemäß Anspruch 3, beispielsweise mit Kegelstumpf- oder Kugelschalenform der Gleitflächen. Dabei kann z.B. durch elastisches Einschnappen der Teile jedes Gleitgelenks ineinander eine Selbstzentrierung und eine dadurch bedingte Selbstfestlegung der Gleitgelenke erreicht werden.

In bestimmten Fällen, wo dies nicht stört, kann ohne große Beeinträchtigung des im Innenraum des Rohres zur Verfügung stehenden Raums oder durch geringfügige Zunahme des Außendurchmessers eine axiale Sicherung der Gleitgelenke auch durch innen oder außen auf das jeweilige Glied aufgebrachte, beliebig dünne Rohrabschnitte oder teilringförmige Rohrteile erfolgen (Anspruch 4). Diese der Sicherung dienenden Teile können natürlich, wenn gewünscht, mehr oder weniger nur Folienstärke besitzen.

Das Überziehen des gesamten Endoskoprohres mit einem elastischen Schlauch gemäß Anspruch 5 kann ebenfalls in einfachster Weise eine Sicherung der Gleitgelenke bewirken, die in diesem Fall sogar als besonders einfach herzustellende und zu montierende Zylindergleitgelenke ausgebildet sein können. Ein derartiger Schlauch kann aus Metallgewebe oder ähnlichen Materialien bestehen.

Der Querschnitt der einzelnen Rohrglieder kann grundsätzlich auch andere als kreisrunde Form haben.

Nach Anspruch 6 sind die rohrförmigen Glieder vorzugsweise aus Metall, Kunststoff oder Sintermaterial hergestellt. Die Herstellung aus Metall gestaltet sich durch Anwendung der Laserschneidtechnik besonders einfach. Aber auch die Herstellung aus Kunststoff läßt sich durch Anwendung der Kunststoff-Spritzgußtechnik sehr einfach gestalten.

Die Ansprüche 7 bis 9 betreffen vorteilhafte Ausgestaltungen für die Führung der Zugseile in der Wandung der Rohrglieder.

Anhand der Figuren werden Ausführungsbeispiele der Erfindung näher erläutert. Es zeigen
- Fig. 1: einen Teilabschnitt einer Ausführungsform des erfindungsgemäßen Endoskoprohres in Schrägansicht,
- Fig. 2: eine vergrößerte Teilschrägansicht des Gelenkkopfes der Gelenkorgane gemäß Fig. 1,
- Fig. 3: eine vergrößerte Teilschrägansicht einer anderen Ausführungsform des in Fig. 2 dargestellten Gelenkkopfes,
- Fig. 4: einen Teilabschnitt einer anderen Ausführungsform des erfindungsgemäßen Endoskoprohrs in Schrägansicht,
- Fig. 5: eine Teilseitenansicht des bei der Ausführungsform gemäß Fig. 4 verwendeten Gelenkorgans,
- Fig. 6: einen Teilabschnitt einer dritten Ausführungsform des erfindungsgemäßen Endoskoprohrs in Schrägansicht,
- Fig. 7: einen Teilschnitt längs der Linie VII-VII in Fig. 4,
- Fig. 8: eine der Fig. 7 entsprechende andere Ausführungsform der in Fig. 7 dargestellten Teile, und
- Fig. 9: einen Teilschnitt längs der Linie IX-IX in Fig. 6.

In allen Figuren sind gleiche oder entsprechende Teile mit den gleichen Bezugszeichen versehen.

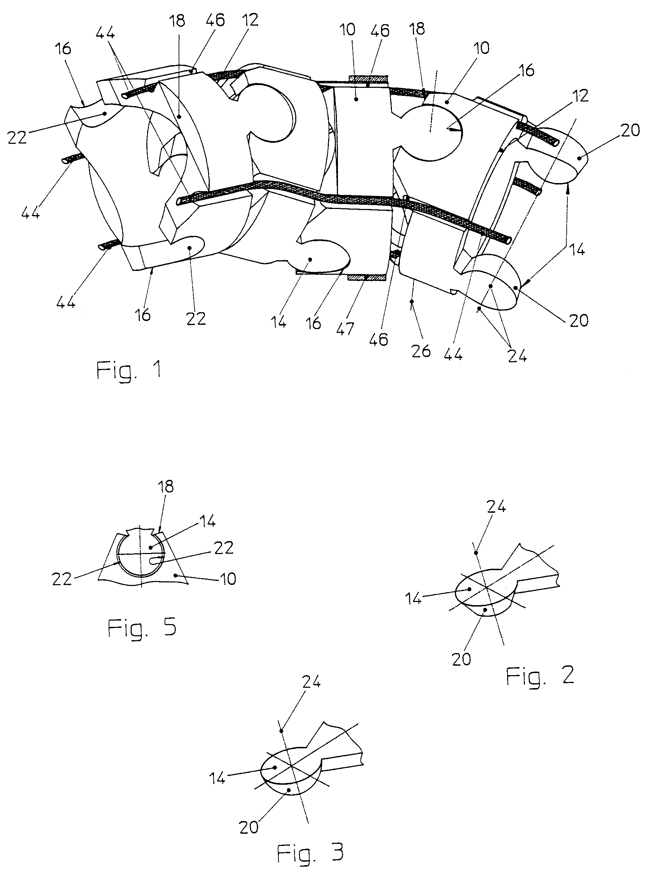

Bei der in den Figuren 1 und 2 gezeigten Ausführungsform weist jedes rohrförmige Glied 10 an diametral einander gegenüberliegenden Stellen je einen aus der einen Stirnfläche 12 des Gliedes 10 etwa senkrecht vorstehenden Gelenkkopf 14 auf, der in je eine entsprechende Gelenkpfanne 16 eingreift, die ausgehend von der gegenüberliegenden Stirnfläche 18 in die Wandung des rohrförmigen Gliedes 10 eingearbeitet ist. Sowohl die Gelenkköpfe 14 als auch die Gelenkpfannen 16 weisen kegelstumpfförmige oder bei der Ausführungsform der Fig. 3 kugelkalottenförmige Gleitflächen 20 bzw. 22 auf, deren Breite in Radialrichtung des Gliedes etwa der Wandstärke des Gliedes 10 entspricht. Paare von Gelenkköpfen 14 und Gelenkpfannen 16 eines Gliedes 10 sind jeweils um 90° gegeneinander verdreht, so daß die zugehörigen Schwenkachsen 24 bzw. 26 aufeinander senkrecht stehen. Dadurch ist eine Biegung des so gebildeten Endoskoprohres in zwei aufeinander senkrecht stehenden Richtungen möglich. Bei einem anderen, nicht dargestellen Ausführungsbeispiel können die Gelenke auch in derselben Ebene liegen. Die kegelstumpfförmige Ausbildung der Gleitfläche 20 eines Gelenkkopfes 14 ist aus Fig. 2 ersichtlich, und die kugelkalottenförmige Ausbildung der Gleitfläche 20 eines Gelenkkopfes 14 ist aus Fig. 3 ersichtlich. Die Gleitflächen 22 der zugehörigen Gelenkpfannen 16 entsprechen jeweils genau den Gleitflächen 20. Bei diesen Ausführungsformen müssen die Gelenkköpfe 14 eine gewisse Elastizität aufweisen, damit sie in die jeweiligen Gelenkpfannen 16 eingeschnappt werden können. Durch ihre sich verjüngende kegelstumpfförmige oder kugelkalottenförmige Ausbildung sind sie nach dem Einschnappen in die Gelenkpfannen in axialer Richtung, in Richtung der Schwenkachsen 24 bzw. 26, festgelegt.

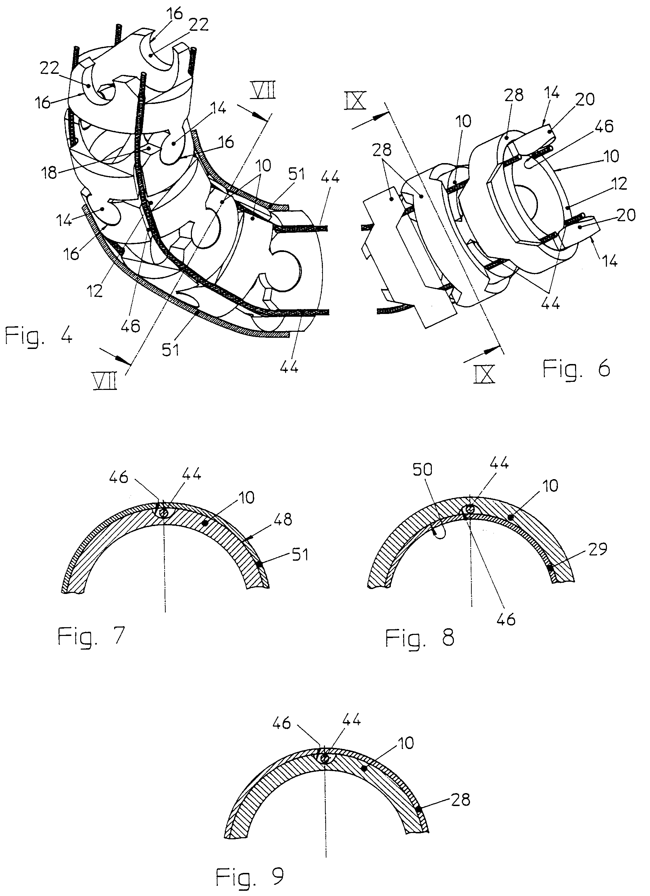

In Anwendungsfällen, bei denen eine sehr geringe Durchmesservergrößerung des Endoskoprohres keine Rolle spielt und daher in Kauf genommen werden kann, ist es möglich, die Gleitflächen 20, 22 der Gelenkorgane 14, 16 gemäß den Figuren 4 bis 6 zylindrisch auszubilden, was herstellungsmäßig natürlich besonders einfach ist, beispielsweise mittels Laserschneidtechnik. Da in diesem Falle jedoch nicht automatisch eine axiale Festlegung der Gelenkorgane 14, 16 erfolgt, wird, wie in Fig. 4 gezeigt, entweder über die Außenseite des ganzen Endoskoprohrs ein dünner elastischer Schlauch 51 gezogen, oder, wie in Fig. 6 gezeigt, auf die Außenseite jedes rohrförmigen Gliedes 10 ein ringförmiges Rohrteil 28 formschlüssig aufgeschoben, welches die Gelenkorgane 14, 16 axial festlegt.

Erfindungsgemäß werden auch die für ein Krümmen oder Verschwenken des Endoskoprohres oder wenigstens dessen vorderen Endes erforderlichen Zugseile innerhalb der Wandstärke der Glieder 10 untergebracht.

Gemäß Figuren 1, 4, 6, 7 und 9 ist jedes Zugseil 44 in einer in die Außenseite 48 jedes Gliedes 10 eingearbeiteten Nut 46 untergebracht. Gemäß Fig. 1 werden die Zugseile 44 in den Nuten 46 durch im Bereich jedes Gliedes 10 auf deren Außenseite aufgebrachte dünne Streifen 47 gehalten, von denen in Fig. 1 nur einer dargestellt ist. Bei der Ausführungsform gemäß Fig. 4 und 7 ist, wie oben bereits erwähnt, ein durchgehender, dünner, elastischer Schlauch 51 über die ganze Außenseite des Endoskoprohrs gezogen, der sowohl die Gelenkorgane 14, 16 in ihren gegenseitigen Lagen radial zum Glied 10 als auch die Zugseile 44 in den Nuten 46 hält. Bei der Ausführungsform gemäß den Figuren 6 und 9 übernehmen die Funktion des Schlauches 51 die auf jedes Glied 10 aufgeschobenen ringförmigen Rohrteile 28.

Bei der abgeänderten Ausführungsform gemäß Fig. 8 sind die die Zugseile 44 führenden Nuten 46 an der Innenseite 50 jedes Gliedes 10 angebracht und innen durch ein sehr dünnes, etwa Folienstärke aufweisendes Rohrteil 29 abgedeckt.

Grundsätzlich ist es auch möglich, die Zugseile 44 in Bohrungen aufzunehmen, die in Längsrichtung des Endoskoprohres durch jedes Glied verlaufen. Dadurch ergäbe sich die geringste radiale Abmessung, die nicht einmal um die Stärke einer Abdeckfolie oder eines folienartigen Schlauches über die Wandstärke der Glieder hinausgeht.

## Patentansprüche

1. Flexibles Endoskoprohr, welches aus kurzen rohrförmigen, durch Schwenkorgane in Längsrichtung des Rohres stabil miteinander verbundenen Gliedern besteht und durch ein oder mehrere längs des Rohres verlaufende Zugseile steuerbar ist, dadurch gekennzeichnet, daß die Schwenkorgane (14, 16) und das bzw. die Zugseile (44) im Bereich jedes rohrförmigen Gliedes (10) innerhalb der Wandstärke des Gliedes (10) angeordnet ist bzw. sind.

2. Endoskoprohr nach Anspruch 1, dadurch gekennzeichnet, daß die Schwenkorgane (14, 16) als Paare von einander diametral gegenüberliegenden, axial festgelegten Gleitgelenken ausgebildet sind.

3. Endoskoprohr nach Anspruch 2, dadurch gekennzeichnet, daß zur axialen Festlegung der Gleitgelenke die Gleitflächen (20, 22) jedes Paares von diametral gegenüberliegenden Schwenkorganen (14, 16) sich in entgegengesetzter Richtung verjüngen.

4. Endoskoprohr nach Anspruch 2, dadurch gekennzeichnet, daß zur axialen Festlegung der Gleitgelenke an der Innen- oder Außenseite jedes Gliedes (10) ein die Gleitgelenke ganz oder teilweise überdeckendes Rohrteil (28) anliegt.

5. Endoskoprohr nach Anspruch 2, dadurch gekennzeichnet, daß zur axialen Festlegung der Gleitgelenke das Endoskoprohr von einem durchgehenden elastischen Schlauch (51) umfaßt ist.

6. Endoskoprohr nach einem der vorangehenden Ansprüche, dadurch gekennzeichnet, daß die rohrförmigen Glieder (10) aus Metall, Kunststoff oder Sintermaterial hergestellt sind.

7. Endoskoprohr nach einem der vorangehenden Ansprüche, dadurch gekennzeichnet, daß die Zugseile (44) in oder an der Innen- oder Außenseite (50 bzw. 48) der rohrförmigen Glieder (10) angeordneten Nuten (46) geführt sind.

8. Endoskoprohr nach Anspruch 7, dadurch gekennzeichnet, daß zur Sicherung der Zugseile (44) die Nuten (46) von einem dieselben ganz oder teilweise überdeckenden, mit dem jeweiligen rohrförmigen Glied (10) fest verbundenen Rohrteil (28) überdeckt sind.

9. Endoskoprohr nach Anspruch 8, dadurch gekennzeichnet, daß die an der Außenseite (48) der rohrförmigen Glieder (10) angeordneten Nuten (46) von einem elastischen Schlauch (51) abgedeckt sind.
